# EUROPÄISCHE PATENTSCHRIFT

(11) **EP 0 550 117 B1**
(45) Veröffentlichungstag und Bekanntmachung des Hinweises auf die Patenterteilung: **15.05.1996**
(21) Anmeldenummer: 92250362.8
(22) Anmeldetag: 22.12.1992
(51) Int. Cl.: A61F 2/32

(54) **Schaftprothese**
Shankprosthesis
Prothèse de tige

(30) Priorität: 30.12.1991 DE 4143288
(43) Veröffentlichungstag der Anmeldung: 07.07.1993
(73) Patentinhaber: ARTOS Medizinische Produkte GmbH, D-12277 Berlin (DE)
(72) Erfinder: Kranz, Curt, Dr.-Ing., W-1000 Berlin 62 (DE)
(74) Vertreter: Christiansen, Henning, Dipl.-Ing.

(56) Entgegenhaltungen:
- EP-A- 0 065 481

## Beschreibung

Die Erfindung betrifft eine Schaftendoprothese der im Oberbegriff des Anspruchs 1 angegebenen Art.

Der Schaft einer derartigen Prothese wird bei der Implantation in einen mit geringfügigem Untermaß vorgeraspelten Knochenhohlraum entweder durch Einzementieren oder durch Preßsitz fixiert.

Aus der DE 29 33 237 A1 ist eine Prothese der vorgenannten Gattung bekannt, die bezüglich ihrer lokalen Elastizität und Steifigkeit an die umgebenden Bereiche des Knochens angepaßt ist. Diese Anpassung bezieht sich aber lediglich auf die Querschnittsabmessungen und ihre Abstimmung auf in Richtung der Längsachse wirkenden Kräfte. Querschnitt und Wandstärke nehmen vom oberen zum unteren Ende des Prothesenschaftes kontinuierlich ab.

Nachteilig ist dabei, daß die bei außerhalb der Längsachse angreifenden Kräften auftretenden Verformungen sich nicht konform zu den entsprechenden Verformungen des angrenzenden Knochenbereichs verhalten. Dadurch kann infolge von relativen Mikrobewegungen eine lokale Lockerung der Prothese erfolgen.

Aus der DE 38 44 157 A1 ist eine weitere Hüftgelenkendoprothese bekannt, die einen sich sowohl bezüglich seines Durchmessers als auch hinsichtlich seinem Materialquerschnitt verjüngenden Hohlschaft aufweist. Dabei nimmt die Längssteifigkeit des Schaftes vom gelenknahen zum gelenkfernen Ende hin annähernd linear ab, während die Biegesteifigkeit des Schaftes zunächst im gelenknahen Bereich nur geringfügig abnimmt, um dann im gelenkfernen Bereich stärker abzunehmen. Somit weist die Endoprothese nur in den Bereichen der hauptsächlichen Krafteinleitung eine hohe Biegesteifigkeit - in den anderen Bereichen hingegen eine verringerte Biegesteifigkeit auf. Dies wird entsprechend chend der vorgeschlagenen Lösung dadurch erreicht, daß zur erheblichen Verringerung der Biegesteifigkeit im gelenkfernen Bereich mindestens ein sich bis zum gelenkfernen Ende hin erstreckender Bereich, an der Innenseite der Krümmung des Prothesenschaftes gelegen ist, ausgespart ist.

Nachteilig bei dieser Endoprothese ist aber, daß ein relativ großer Oberflächenbereich der Endoprothese, im proximalen Bereich der lateralen Seite des Prothesenschafts, an der die Belastung nicht vom Prothesenschaft auf den Knochen übertragen wird, mit dem Knochen verbunden ist. Da die Biegesteifigkeit des Prothesenschafts in diesem Bereich noch relativ hoch und nur annäherungsweise an diejenige des Knochens angepaßt ist, treten hier Schubkräfte auf, die aus der unterschiedlichen Verformung von Prothesenschaft und Knochen bei Belastung resultieren. Diese Schubkräfte führen zu Verschiebungen an den Grenzflächen und somit möglicherweise auch zur vorzeitigen Lockerung der Endoprothese.

Aus der WO-A-90/02 533 ist eine Schaftprothese der eingangs genannten Gattung bekannt, welche einen seitlich offenen Hohlkörper aufweist. Die Gestaltung der Öffnung ist jedoch nicht mit derjenigen der hier beschriebenen Prothese vergleichbar, da einerseits wegen der durchgehenden Öffnung des Schlitzes die Torsionssteifigkeit des Schaftes stark herabgesetzt ist. Ferner weist sie keine Festigkeitseigenschaften auf, bei der die Biegesteifigkeit einerseits zum gelenkfernen Ende hin in der erwünschten Weise wesentlich stärker abnimmt als die Längssteifigkeit und andererseits aber im Bereich des Adamschen Bogens auf einem hohen Niveau verbleibt.

Der Erfindung liegt die Aufgabe zugrunde, eine Schaftprothese der eingangs genannten Gattung mit an den umgebenden Knochen und die in diesen einzuleitenden Kräfte angepaßter konstruktiver Gestaltung anzugeben, bei der der Innenraum der Prothese von der Gelenkseite her zugänglich gehalten ist.

Diese Aufgabe wird mit den Merkmalen des Anspruchs 1 gelöst.

Durch bei der erfindungsgemäßen Prothese erreichte nur geringe Reduktion der Biegesteifigkeit im proximalen Drittel wird insbesondere die Einleitung der Torsionskräfte verbessert. Durch die wulstige Ausgestaltung der Schlitzränder ist ferner das Rißrisiko reduziert. Durch die gewählte geometrische Ausgestaltung ist in vereinfachter Weise die Möglichkeit des "Ausbohrens" von in den Protheseninnenraum eingewachsenen Knochengewebes gegeben.

Durch die mit der erfindungsgemäßen Prothese erzielbaren elastischen Anpassung der Protheseneigenschaften in Verbindung mit einer Zugänglichkeit des Innenraums bei Reoperationen wird einerseits im distalen Femur eine Sockelbildung verhindert - es entsteht also weniger den Sitz der Prothese beeinträchtigendes Knochenmaterial, andererseits läßt sich aber im Schaftinneren aufgebaute Knochensubstanz bei eventuell notwendigen Reoperationen durch einfache Maßnahmen schnell entfernen.

Weiterhin ist im proximalen Femur eine Resorption durch Reduktion des Stresshieldings mindestens stark verringert, wenn nicht sogar vollständig unterbunden.

Die erfindungsgemäße Schaftprothese weist eine annähernd linear abnehmende Längssteifigkeit und eine nicht linear abnehmende Biegesteifigkeit auf. Die Abnahme der Längssteifigkeit wird erreicht durch eine entsprechende Abnahme des Materialquerschnitts, wobei die Biegesteifigkeit, in die das Flächenträgheitsmoment eingeht, im proximalen Bereich nur langsam abnimmt, um dann im oberen distalen Bereich, bedingt durch die schlitzartige Durchbrechung und die wesentlich kleinere Querschnittsfläche in der medialen Seite, rasch auf einen niedrigen Wert abzusinken, der dann nur noch langsam bis zum distalen Ende des Schafts weiter abnimmt.

Dadurch, daß der Schaft schlitzartige Durchbrechungen aufweist, ist die Oberfläche des Schafts, die mit dem Knochen in Verbindung ist, geringer als bei anderen bekannten Prothesenhohlschäften. Um die Schubbelastung pro Flächeneinheit zu kontrollieren, ist der proximale Bereich des Schafts bei einer vorteilhaften Weiterbildung der Erfindung mit einer besonderen Oberflächengestaltung in Form einer Profilierung versehen, die die Oberfläche im proximalen Bereich vergrößert. Diese Profilierung ist vorzugsweise auf den nach dorsal und/oder ventral gerichteten Oberflächenbereichen des Schafts vorgesehen. Die Profilierung besteht vorzugsweise aus einer Rillenstruktur, die im wesentlichen in Längsrichtung des Schafts gerichtet ist, wobei die einzelnen Rillen der Rillenstruktur als in etwa halbkreisförmige und in die Schaftoberfläche eingelassene Vertiefungen ausgebildet sind.

Bei der Wahl der Rillentiefe und Rillenbreite wurde beachtet, daß einerseits die erforderlichen lokalen Biege- und Längssteifigkeiten mit dem Materialquerschnitt und der Querschnittsfläche des Schafts erzielbar sein müssen und daß andererseits die Oberfläche des Schafts durch die eingelassene Rillenstruktur ausreichend vergrößert wird, um eine zuverlässige Einleitung der Schubbelastung in die banachbarten Knochenbereiche zu erreichen. Das Eintreiben der Prothese wird durch diese Längsrillen nicht beeinträchtigt - sperrend wirken sie hingegen gegen in Richtung des Prothesenhalses auftretende Schubkräfte. Durch die bevorzugte Krafteinleitung im Bereich gegenüberliegender Wandungen im proximalen Femurbereich läßt sich eine bevorzugte metaphysäre Verankerung der Prothese erreichen.

Bei einer günstigen Ausführungsvariante der Erfindung wird die Rillentiefe etwas kleiner als der Rillendurchmesser gewählt, so daß einerseits die Materialabnahme bedingt durch die Rillenstruktur klein gehalten wird und daß andererseits eine Oberflächenzunahme von ca. 20% im Bereich jeder Rille erzielbar ist.

Die schlitzartige Durchbrechung im proximal-lateralen Bereich des erfindungsgemäßen Prothesenschafts ist bevorzugt derart ausgebildet, daß sie sich bis zum Prothesenhalsansatz erstreckt und - in Längsrichtung der Prothese gesehen - einen Querschnitt aufweist, der es ermöglicht, in den Innenraum des Schafts ein Räum- oder Führungsinstrument einzuführen, welches sich in gerader Richtung bis zum distalen Ende der Innenausnehmung erstreckt. Hiermit ist es möglich in den Innenraum der Prothese eingewachsene Knochensubstanz zu entfernen, um im Falle einer Reimplantation ein einfaches Ausziehen des Schafts aus dem Femur zu ermöglichen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine Seitenansicht eines Ausführungsbeispiels der erfindungsgemäßen Schaftprothese,
Figuren 1a bis 1c Querschnitte A-A, B-B und C-C der Schaftprothese gemäß Figur 1,
Figur 2 eine Seitenansicht einer vorteilhaften Weiterbildung der erfindungsgemäßen Schaftprothese, sowie
Figuren 2a und 2b einen Querschnitt B'-B' und eine vergrößerte Darstellung einer Rille der Rillenstruktur der Schaftprothese gemäß Figur 2.

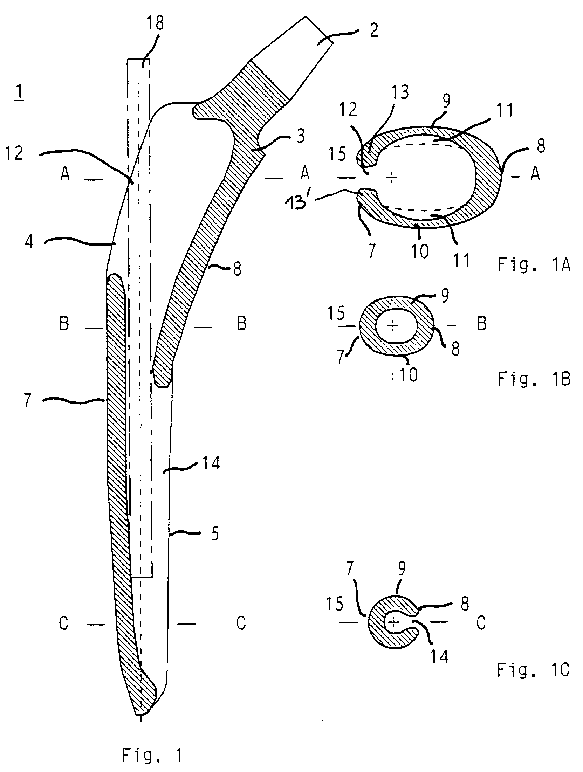

In Figur 1 ist ein Ausführungsbeispiel der erfindungsgemäßen Schaftprothese 1 im Längsschnitt dargestellt, woraus die Verteilung der Wandungsbereiche des Schafts 3 ersichtlich ist. Dieser besteht aus einer sich nach distal hin verjüngenden Röhre, welche verschiedene Aussparungen aufweist, die die Festigkeit des Schaftes beeinflussen bzw. einen Zugang zum Schaftinneren ermöglichen. Der als Ausführungsbeispiel dargestellte Prothesenschaft zur Anwendung im Hüftgelenk weist einen Konus zur Aufnahme einer Gelenkkugel auf. Die dargestellte Prothese besteht aus einer körperverträglichen Titanlegierung und kann durch Schmieden zweier Halbschalen erzeugt werden, welche nachträglich miteinander verschweißt werden.

Aus der Längsschnittdarstellung gemäß Figur 1 und den Querschnitten gemäß Figuren 1A bis 1C ist ersichtlich, daß der Schaft 3 im proximalen Abschnitt 4 des lateral - d.h. in bezug auf die Hauptkrümmung des Schaftes außen - gelegenen Wandungsbereichs 7 und im distalen Abschnitt 5 des medial - in Bezug auf die Hauptkrümmung des Schaftes innen - gelegenen Wandungsbereichs 8 jeweils eine in Längsrichtung des Schafts 3 verlaufende schlitzartige Durchbrechung 12 bzw. 14 aufweist. Die als Ausführungsbeispiel dargestellte Prothese ist symmetrisch, d.h. für und links- und rechtsseitige Implantation geeignet. Die Richtungen "dorsal" und "ventral" sind damit vertauschbar, weil sie sich auf den Einsatz bei einer bestimmten ausgewählten Körperseite beziehen.

Die schlitzartige Durchbrechung 12 im proximalen Abschnitt 4 des lateralen Seitenbereichs 7 erstreckt sich vom unteren Ende des den die Gelenkkugel aufnehmenden Konus 2 tragenden Halses über etwa ein Drittel der Schaftlänge. Die weitere schlitzartige Durchbrechung 14 im distalen Abschnitt 5 des medialen Bereichs 8 erstreckt sich über im wesentlichen die Hälfte der Länge des Schafts 3, wobei sie aber das gelenkferne Ende des Schafts 3 nicht vollständig erreicht, sondern kurz vorher endet, so daß der beim Eintreiben des Schafts vorangeführte Endbereich sich in Eintreibrichtung verjüngend massiv ausgebildet ist und somit beim Eintreiben des Schafts 3 ein Eindringen von Knochenmaterial in das Schaftinnere verhindert ist.

Anhand der Figuren 1a bis 1c soll nun die Ausgestaltung verschiedener repräsentativer Querschnittsbereiche des Schafts 3 näher erläutert werden. In Figur 1a ist der im proximalen Bereich gelegene, in Figur 1 mit A-A bezeichnete Schnitt des Schafts 3 dargestellt. Die Wandstärke des Schafts 3 in den dorsalen und ventralen Seitenbereichen 9 bzw. 10 sind hierbei durch lokale Schwächungen 11 bzw. 11' im Vergleich zu den lateral und medial gelegenen Wandungsbereichen 8 bzw. 13 und 13' verringert. Die Wandungsbereiche 13 und 13' bilden dabei Verdickungen, welche entlang der Durchbrechung 14 verlaufen. Die Ausbildung der Wandstärken im Bereich benachbart zu dieser Durchbrechung (Bereiche 13 und 13') und im gegenüberliegenden Wandungsbereich 8 ist derart gewählt, daß die durch die Durchbrechung verursachte Verringerung der Biegesteifigkeit ausgeglichen ist. Die Querschnitte der Kantenbereiche 13 und 13' sind zur Herabsetzung von Materialspannungen elliptisch verrundet. Entsprechendes gilt auch für die Gestaltung der (in der Zeichnung nicht dargestellten) Endbereiche der schlitzartigen Durchbrechungen.

Die zwischen den der schlitzartigen Durchbrechung 12 benachbarten Kantenbereichen 13 und 13' und dem Wandungsbereich 8 befindlichen dorsalen und ventralen Bereiche 9 bzw. 10 sind demgegenüber in ihrer Wandstärke verringert.

Die erste und zweite schlitzartige Durchbrechung 12 bzw 14 überdecken einander (bezogen auf die Schaftlängsrichtung) nicht. Dies ergibt sich auch aus Figur 1B anhand des Prothesenquerschnitts B-B (gemäß Figur 1). Durch die mit dem stark verringerten Flächenträgheitsmoment hier bereits stark verringerte Biegesteifigkeit der Prothese endet der Bereich der maximalen Einleitung von Schubkräften über die dorsal und ventral gelegenen geschlossenen Flächenbereiche im proximalen Bereich 4 des Schafts. Auch sind hier die lateral und medial gelegenen Wandungsbereiche 7 und 8 nur noch wenig bzw. gar nicht mehr im Vergleich zu den dorsal und ventral gelegenen Bereichen verstärkt.

Anschließend daran verringert sich die Querschnittsfläche weiterhin bis zum distalen Ende, wobei die zweite schlitzartige Durchbrechung 14 im medialen Wandungsbereich 8, die nahezu bis zur Schaftspitze verläuft, anhand der Figur 1C (Schnitt C-C gemäß Figur 1) näher erkennbar ist. Die Biegesteifigkeit des Schafts ist durch die Durchbrechung 14 zusätzlich herabgesetzt, wobei hier bewußt kein Ausgleich durch Heraufsetzung der Wandstärke in den medial bzw. lateral gelegenen Wandungsbereichen erfolgte.

Wie aus den Schnitten gemäß Figuren 1A bis 1C ersichtlich, ist der Schaft zur Lateral-Medial-(LM-)Ebene symmetrisch, wobei diese Ebene die Zeichenebene in der Symmetrieachse der Figuren schneidet. Es ist gleichzeitig diejenige Ebene, in der die der maximalen Krümmung folgende Mittellinie der Prothese verläuft. Die Querschnittsform und damit auch der Materialquerschnitt und die daraus resultierenden Festigkeitseigenschaften des Schafts sind zur dorsalventralen Ebene 15 symmetrisch, so daß sich die Schaftprothese 1 sowohl zur links- als auch zur rechtsseitigen Implantation eignet.

Ein in der Figur 1 gestrichelt dargestelltes Instruments 18 kann in die schlitzartige Durchbrechung 12 der erfindungsgemäßen Schaftendoprothese 1, die sich bis zum Prothesenhals 2 erstreckt, von oben in den Schaft 3 eingeführt werden. Das Instrument 18 ist derart dimensioniert, daß es bis in den distalen Bereich 5 des Schafts 3 einführbar ist. Von der dicker ausgebildeten Wandung der lateralen Seite 7 beidseitig der schlitzartigen Durchbrechung 12 wird das Instrument 18 sicher gehalten. Wenn die erfindungsgemäße Schaftprothese 1 in die vorbereitete Knochenhöhlung eingesetzt wird, ist sie dann, durch das Instrument 18, führbar. Mit einem entsprechend geformten Räuminstrument kann der Protheseninnenraum erreicht werden, um durch die Durchbrechung 14 eingewachsenes Knochengewebe vor dem Entfernen des Prothesenschaftes im Falle einer Reoperation zu beseitigen.

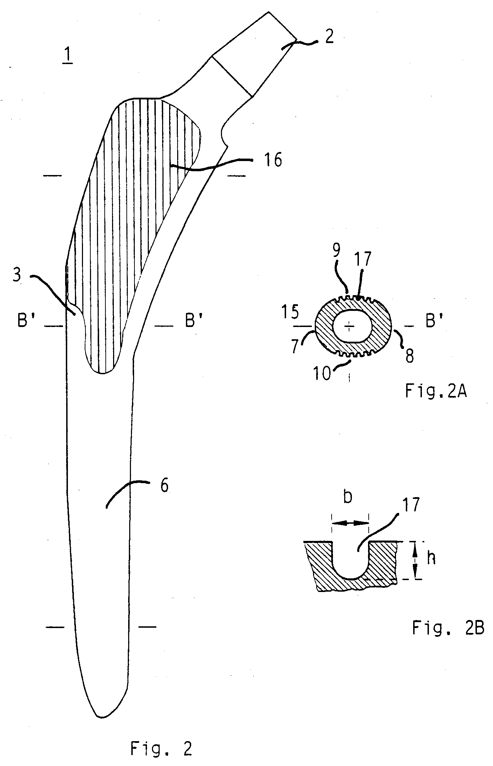

In Figur 2 ist das in den Figuren 1 bis 1C dargestellte Ausführungsbeispiel in einer mit einer Profilierung der Außenoberfläche versehenen Variante in seiner Außengestaltung in Seitenansicht dargestellt. Die Schaftoberfläche 6 ist im proximalen und gekrümmten Bereich 4 nach dorsal und ventral hin mit einer Profilierung 16 versehen. Sie ist an der dorsalen und ventralen Wandungbereichen 9 bzw. 10 des Schafts 3 ange-bracht und erstreckt sich im Bereich der schlitzartigen Durchbrechung 12 in der lateralen Seite 7 bis zum Anfang Druchbrechung 14. Im übrigen setzt sich die Profilierung 15 an den lateralen und medialen Wandungsbereichen 7 bzw. 8 nicht fort, wie aus dem in Figur 2A dargestellten Prothesenquerschnitt (B'-B' in Figur 2) ersichtlich ist.

Es ist ersichtlich, daß die Oberflächenprofilierung derart gerichtet ist, daß sie keine sperrende Wirkung in Längs-(d.h. in Eintreibrichtung) der Prothese ausübt, hingegen aber in der Lage ist, große Schubkräfte in Richtung der Längsachse des Prothesenhalses (entsprechend der Längsachse des die Gelenkkugel aufnehmenden Schauftkonus) in die angrenzenden Femurbereiche einzuleiten. Dem sich dabei einstellenden Kraftfluß entspricht die Bemessung der Biege-und Längssteifigkeit des Schaftes. Die Biegesteifigkeit ist durch die beschriebenen konstruktiven Maßenahmen in Richtung zum distalen Ende des Schaftes dort wesentlich herabgesetzt, wo die Profilierung endet.

Wie ersichtlich, ist der Materialquerschnitt - und damit auch die konstruktive Auslegung hinsichtlich der Festigkeits des Schafts 3 auch bei dieser Variante symmetrisch zur A-P-Ebene 15.

Anhand von Figur 2B (als Vergrößerung der Darstellung gemäß Figur 2A) wird die genauere Dimensionierung anhand einer vergrößerten Wiedergabe einer einzelnen Rille 17 näher dargestellt. Die Rille 17 weist eine Breite b auf, die geringfügig größer ist als deren Tiefe h, mit der die Rille 17 in die Schaftoberfläche 6 eingelassen ist. Der Grund der Rille weist einen im wesentlichen halbkreisförmig verrundeten Querschnitt auf.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Schaftendoprothese (1), insbesondere Hüftgelenkprothese, mit einem im wesentlichen rohrförmig sich verjüngenden und im proximalen Bereich gekrümmten Schaft (3), bei dem vom gelenknahen zum gelenkfernen Ende hin die Biegesteifigkeit stärker abnimmt als die Längssteifigkeit, mit einer sich in Längsrichtung des Schafts (3) erstreckenden schlitzartigen ersten Durchbrechung (12), welche als Ausnehmung die Fortsetzung des Innenraums des distalen Endbereichs des Schaftes in gerader Richtung bildet, wobei mindestens eine der Seitenkanten (13, 13') der schlitzartigen ersten Durchbrechung (12) eine im Vergleich zu dem sich in tangentialer Richtung anschließenden Wandungsteil verdickte Wandstärke aufweist, in der Weise, daß die durch die schlitzartige erste Durchbrechung (12) verursachte Verringerung der Längs- und/oder Biegesteifigkeit im wesentlichen ausgeglichen ist,
**dadurch gekennzeichnet**, daß
die Seitenkanten (13, 13') der schlitzartigen ersten Durchbrechung (12) eine im Vergleich zu dem sich in tangentialer Richtung anschließenden Wandungsteil verdickte Wandstärke aufweisen, und daß die erste Durchbrechung (12) nur in einem auf dem Rücken der Krümmung gelegenen Bereich vorgesehen ist und eine sich an der Innenseite der Schaftkrümmung in Längsrichtung des Schaftes bis in die Nähe des proximalen Endes erstreckende zweite Durchbrechung (14) vorgesehen ist.

2. Schaftendoprothese nach Anspruch 1, **dadurch gekennzeichnet,** daß mindestens im proximalen Bereich des Schafts (4) die lateralen und medialen Wandungsbereiche (7, 8) dicker ausgebildet sind als die dorsal bzw. ventral gelegenen Wandungsbereiche (9, 10).

3. Schaftendoprothese nach Anspruch 1 oder 2, **dadurch gekennzeichnet,** daß die Projektionen der ersten und der zweiten Durchbrechung (12 bzw. 14), in lateral-medialer Richtung gesehen, einander nicht überdecken.

4. Schaftendoprothese nach Anspruch 3, **dadurch gekennzeichnet,** daß der Bereich, in dem sich die erste und die zweite Durchbrechung (12 bzw. 14) nicht überdecken, in einem Übergangsbereich zwischen einem gekrümmten (proximalen) und einem im wesentlichen gerade gerichteten (distalen) Abschnitt des Schafts (3) gelegen ist.

5. Schaftendoprothese nach einem der vorangehenden Ansprüche, **dadurch gekennzeichnet,** daß distale Ende des Schafts (3) sich zum Ende hin verjüngend und verrundet massiv ausgebildet ist.

6. Schaftendoprothese nach einem der vorangehenden Ansprüche, **dadurch gekennzeichnet,** daß die erste schlitzartige Durchbrechung (12) im proximalen Bereich der lateralen Seite (7) derart ausgebildet ist, daß zur Führung der Schaftendoprothese (1) bei der Implantation in die Knochenaushöhlung ein Instrument (18) von oben in die schlitzartige Durchbrechung (12) einsteckbar ist und von der dicker ausgebildeten Wandung der lateralen Seite (7) beidseitig der schlitzartigen Durchbrechung (12) sicher gehalten wird.

7. Schaftendoprothese nach einem der vorangehenden Ansprüche, **dadurch gekennzeichnet,** daß die Prothese zur eine maximale Krümmung aufweisenden Mittelebene des Schafts (3) symmetrisch ausgebildet ist, so daß die Implantation sowohl links- als auch rechtsseitig erfolgen kann.

8. Schaftendoprothese nach einem der vorangehenden Ansprüche, **dadurch gekennzeichnet,** daß die dorsalen und ventralen Oberflächen (9, 10) des Schafts (3) mindestens teilweise mit sich im wesentlichen in Längsrichtung des Schafts (3) erstreckenden Rillen (16) versehen sind.

9. Schaftendoprothese nach Anspruch 8, **dadurch gekennzeichnet,** daß die einzelnen Rillen (17) der Rillenstruktur (16) als in etwa halbkreisförmige und in die Schaftoberfläche (6) eingelassene Vertiefungen ausgebildet sind.

## Claims

1. A shank endoprosthesis (1), more particularly a hip joint prosthesis, comprising a substantially tubularly tapering shank (3) which is curved in the proximal region, in which shank the flexural stiffness decreases more than the longitudinal stiffness proceeding from the end near the joint to the end remote therefrom, a longitudinally extending, slot-like first perforation (12) which rectilinearly continues, as a cut-out portion, the inner space of the distal end region of the shank, at least one of the side edges (13, 13') of the slot-like, first perforation (12) having a greater wall thickness than the wall section adjoining said edges in the tangential direction, so that the decrease in the longitudinal and/or flexural stiffness caused by the slot-like, first perforation (12) is largely compensated, characterised in that the side edges (13, 13') of the slot-like first perforation (12) have a greater wall thickness than the wall section adjoining said edges in the tangential direction, and that the first perforation (12) is only provided in a region arranged on the outside of the curvature, and that a second perforation (14) is provided on the inside of the curvature of the shank, said second perforation extending in the longitudinal direction of the shank up to the vicinity of the proximal end.

2. A shank endoprosthesis according to claim 1, characterised in that at least in the proximal region of the shank (4) the lateral and medial wall sections (7, 8) are of a greater thickness than the dorsal or ventral wall regions (9, 10).

3. A shank endoprosthesis according to claim 1 or 2, characterised in that the projections of the first and second perforations (12 and 14) do not overlap, viewed in the lateral-medial direction.

4. A shank endoprosthesis according to claim 3, characterised in that the region in which the first and second perforations (12 and 14) do not overlap is positioned in a transitional region between a curved (proximal) and a substantially straight (distal) portion of the shank (3).

5. A shank endoprosthesis according to one of the preceding claims, characterised in that the distal end of the shank (3) is of a solid, rounded construction, tapering to its end.

6. A shank endoprosthesis according to one of the preceding claims, characterised in that the slot-like, first perforation (12) is formed in the proximal region of the lateral side (7) so that, in order to guide the shank endoprosthesis (1) during implantation into the bone cavity, an instrument (18) may be inserted from above into the slot-like perforation (12) and is reliably held by the thicker wall of the lateral side (7) on both sides of the slot-like perforation (12).

7. A shank endoprosthesis according to one of the preceding claims, characterised in that the prosthesis is symmetrically constructed about a central plane of the shank (3), which comprises a maximum curvature, so that implantation may be carried out both on the left-and right-hand side.

8. A shank endoprosthesis according to one of the preceding claims, characterised in that the dorsal and ventral surfaces (9, 10) of the shank (3) are provided, at least partially, with grooves (16) extending substantially in the longitudinal direction of the shank (3).

9. A shank endoprosthesis according to claim 8, characterised in that the individual grooves (17) of the groove structure (16) are in the form of substantially semi-circular recesses provided in the shank surface (6).

## Revendications

1. Endoprothèse à tige (1), en particulier prothèse d'articulation de la hanche, comportant une tige (3) sensiblement tubulaire, qui va en se rétrécissant et qui est coudée dans la région proximale, dans laquelle la rigidité à la flexion diminue plus fortement, depuis l'extrémité près de l'articulation jusqu'à l'extrémité éloignée de l'articulation, que la rigidité longitudinale, comportant une première traversée (12) en forme de fente s'étendant en direction longitudinale de la tige (3), qui forme en tant qu'évidement le prolongement de la chambre intérieure de la région finale distale de la tige en direction linéaire, l'une au moins des arêtes latérales (13, 13') de la première traversée (12) en forme de fente présentant une épaisseur de paroi plus épaisse par comparaison à la partie de paroi s'étendant en direction tangentielle, de telle sorte que la réduction de la rigidité longitudinale et/ou à la flexion, qui est causée par la première traversée (12) en forme de fente est sensiblement compensée, caractérisée en ce que les arêtes latérales (13, 13') de la première traversée (12) en forme de fente présentent une épaisseur de paroi plus épaisse par comparaison à la partie de paroi qui se raccorde en direction tangentielle, et en ce que la première traversée (12) est prévue seulement dans une région située sur le dos de la courbure, et en ce qu'il est prévu une secondè traversée (14) s'étendant sur le côté intérieur de la courbure de tige en direction longitudinale de la tige jusqu'à proximité de l'extrémité proximale.

2. Endoprothèse à tige selon la revendication 1, caractérisée en ce qu'au moins dans la région proximale de la tige (4), les régions de paroi latérales et médiales (7, 8) sont réalisées de façon plus épaisse que les régions de paroi (9, 10) situées en direction dorsale ou ventrale.

3. Endoprothèse à tige selon l'une ou l'autre des revendications 1 et 2, caractérisée en ce que les projections de la première et de la seconde traversées (12 ou 14) ne se chevauchent pas, vues en direction latérale-médiane.

4. Endoprothèse à tige selon la revendication 3, caractérisée en ce que la région dans laquelle la première et la seconde traversée (12 ou 14) ne se chevauchent pas est située dans une région de transition entre un tronçon coudé (proximal) et un tronçon dirigé sensiblement linéairement (distal) de la tige (3).

5. Endoprothèse à tige selon l'une quelconque des revendications précédentes, caractérisée en ce que l'extrémité distale de la tige (3) est réalisée de manière massive et arrondie en rétrécissement vers l'extrémité.

6. Endoprothèse à tige selon l'une quelconque des revendications précédentes, caractérisée en ce que la première traversée (12) en forme de fente est réalisée dans la région proximale du côté latéral (7), de telle sorte que pour le guidage de l'endoprothèse à tige (1) lors de l'implantation dans la cavité de l'os, un instrument (18) peut être enfilé depuis le haut dans la traversée (12) en forme de fente, et est retenu de façon sûre par la paroi réalisée de façon plus épaisse du côté latéral (7) des deux côtés de la traversée (12) en forme de fente.

7. Endoprothèse à tige selon l'une quelconque des revendications précédentes, caractérisée en ce que la prothèse est réalisée de façon symétrique par rapport au plan médian de la tige (3), qui présente une courbure maximum, de sorte que l'implantation peut s'effectuer tant du côté gauche que du côté droit.

8. Endoprothèse à tige selon l'une quelconque des revendications précédentes, caractérisée en ce que les surfaces dorsale et ventrale (9, 10) de la tige (3) sont pourvues au moins partiellement de rainures (16) s'étendant sensiblement en direction longitudinale de la tige (3).

9. Endoprothèse à tige selon la revendication 8, caractérisée en ce que les rainures individuelles (17) de la structure de rainure (16) sont réalisées sous forme de renfoncements approximativement demi-circulaires et ménagés dans la surface (6) de la tige.
